# FASCICULE DE BREVET EUROPEEN

(11) **EP 0 368 758 B1**
(45) Date de publication et mention de la délivrance du brevet: **20.07.1994**
(21) Numéro de dépôt: 89403085.7
(22) Date de dépôt: 08.11.1989
(51) Int. Cl.: C07F 3/00, C07C 309/18, A61K 31/315

(54) **Le cystéate de zinc, sa préparation et ses applications pharmaceutiques et cosmétologiques**
Zinncysteat, dessen Herstellung und dessen pharmazeutische und kosmetische Verwendungen
Zinc cysteate, its preparation and its pharmaceutical and cosmetic uses

(30) Priorité: 09.11.1988 FR 8814909
(43) Date de publication de la demande: 16.05.1990
(73) Titulaire: LABORATOIRES PHARMACEUTIQUES ROCHE-POSAY, 86270 La Roche-Posay (FR); CHIMIE et BIOLOGIE, F-92700 Colombes (FR)
(72) Inventeur: Blum, Jean, F-92400 Courbevoie (FR); Guillard, Olivier, F-86000 Poitiers (FR)
(74) Mandataire: Blum, Jean

(56) Documents cités:
- EP-A- 0 062 362
- FR-A- 2 241 301
- GB-A- 991 552

## Description

Le domaine de la présente invention est celui de la biochimie du zinc et de ses applications pharmaceutiques et cosmétologiques.

On a constaté l'existence de cet oligo-élément dans les milieux biologiques depuis plus d'un siècle et l'ouvrage de Jean LEDERER, "Le zinc en
logie et en Biologie" aux éditions Nauwelaerts-Bruxelles 1985, résume les connaissances actuelles sur le sujet.

L'un des co-inventeurs de la présente invention a étudié le métabolisme d'un nouveau pantothéate de zinc (O.GUILLARD-Thèse-Doctorat d'Etat en Pharmacie-Université de Poitiers 1978), et il est bien connu que le sel de zinc de la pyridine-2-thione est le principe actif de nombreuses lotions antipelliculaires.

Le zinc est indispensable pour la croissance et la multiplication cellulaire et la carence en zinc handicape la cicatrisation des plaies.Cependant, A.FAVIER et collaborateurs (Lyon Pharmaceutique 1980 31(6)357-366) soulignent que l'apport en zinc chez un animal normal n'augmente pas la cicatrisation.

Il est donc tout à fait surprenant que le nouveau sel objet de la présente invention, le cystéate de zinc, ait des propriétés cicatrisantes, et ce d'autant plus que la partie organique du sel, l'acide cystéïque, n'est pas connue pour cette application thérapeutique puisque sa fonction soufrée se trouve sous forme oxydée .

La présente invention concerne ainsi, sous forme anhydre ou hydratée, le cystéate de zinc de formule suivante: 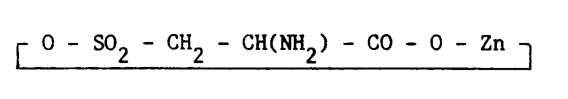
La présente invention concerne tout particulièrement la forme dihydratée du sel de zinc de l'acide L-cystéïque de formule brute C₃H₅O₅ NS Zn,2 H₂O qui est dénommée dans ce qui suit par le nom de code CB 619.

Le CB 619 se présente sous forme d'une poudre blanche cristalline, de saveur astringente que l'on conserve à l'abri de la lumière et dans un récipient bouché. Il est insoluble dans les solvants organiques courants, mais soluble dans le diméthylsulfoxyde et dans l'eau jusqu'à une concentration voisine de 6% poids par volume. Le pH de la solution à 6% est d'environ 6,30.

En solution à 0,1% dans HCl 0,1 N son spectre U.V. présente un maximum d'absorption à environ 197 nm. Le spectre I.R. présente de larges bandes à 3 500 et 3 400 cm⁻¹ , et on y retrouve les bandes des groupements C=O à 1 600 et 1 200 cm⁻¹, -O-SO₂- à 1 400 et 1 100 cm⁻¹ et S=O à 900 cm⁻¹.

La présente invention concerne également les procédés de fabrication du cystéate de zinc qui consistent à faire réagir l'acide cystéïque avec un sel, un oxyde ou hydroxyde de zinc de préférence en milieu aqueux pour des raisons d'économie. Suivant l'invention, on peut remplacer l'acide cystéîque par l'un de ses sels, de préférence soluble dans l'eau.

Suivant d'autres procédés de l'invention, on obtient le cystéate de zinc par action du peroxyde de zinc sur la cystine ou la cystéine en milieu légèrement acide , par exemple en utilisant les chlorhydrates de cystine ou de cystéïne. Cependant le cystéate de zinc obtenu est moins pur que celui obtenu selon les procédés précédemment décrits, et pour obtenir un sel pharmaceutiquement acceptable, on doit le purifier, par exemple par plusieurs recristallisations.

Les nouveaux procédés de fabrication du cystéate de zinc suivant l'invention seront mieux compris à la lecture des exemples suivants donnés à titre non limitatif .

### EXEMPLE 1: Préparation à partir du sulfate de zinc.

On dissout 3,744 g d'acide L-cystéïque monohydraté dans 55,94 ml d'une solution de baryte 0,715 N. D'autre part, on dissout 3,588 g de sulfate de zinc monohydraté dans 50 ml d'eau distillée. On mélange les deux solutions ce qui fait précipiter le sulfate de baryum. Celui-ci est séparé par filtration et lavé deux fois avec 10 ml d'eau. On rassemble les filtrats et on les évapore sous vide. On obtient ainsi le CB 619 brut qui est recristallisé dans deux fois son poids d'eau, lavé avec 5 ml de méthanol et séché à l'étuve à 30-40°C.

### EXEMPLE 2: Préparation à partir de l'oxyde de zinc.

On dissout 16,92 d'acide L-cystéïque anhydre dans 200 ml d'eau distillée et ajoute 8,14 g d'oxyde de zinc. On chauffe à ébullition, ce qui provoque la solubilisation quasi parfaite du mélange. On ajoute un peu de noir décolorant et on filtre la solution. Le filtrat est évaporé sous vide jusqu'à obtention d'un volume d'environ 100 ml et on laisse cristalliser le CB 619 qui est essoré, lavé deux fois avec 20 ml de méthanol et séché à l'étuve à 30-40°C.

### EXEMPLE 3: Préparation à partir du carbonate de zinc.

ON dissout partiellement en agitant 550 g d'acide L-cystéïque anhydre dans deux litres d'eau distillée et on ajoute 510 g de carbonate de zinc en poudre tout en agitant. On chauffe ainsi à ébullition ce qui conduit à une solution relativement limpide à laquelle on ajoute un peu de noir décolorant. On filtre à chaud le mélange. On refroidit le filtrat à 0-5°C et on essore sur filtre le CB 619 brut qui a cristallisé. Celui-ci est recristallisé comme précédemment dans deux fois son poids d'eau, lavé deux fois au méthanol et séché à l'étuve à 30-40°C.

### EXEMPLE 4: Préparation à partir du peroxyde de zinc et le chlorhydrate de cystéïne.

On dissout 17,55 de chlorhydrate de cystéïne monohydraté dans 250 ml d'eau et on ajoute 70 g de peroxyde de zinc en agitant. On obtient une pâte qui est agitée à 30-35°C pendant 4 heures. On sépare alors le produit en suspension par filtration. Le gâteau est lavé deux fois avec 50 ml d'eau. Les filtrats sont rassemblés et évaporés sous vide jusqu'à environ 60 ml. En refroidissant à 0-5°C, le CB 619 brut précipite. Il doit être recristallisé eu moins deux fois pour obtenir un produit de même pureté que dans les exemples 1, 2 et 3 ci-dessus. On le lave au méthanol et on le sèche comme précédemment mentionné.

La présente invention a aussi pour objet les applications pharmaceutiques et notamment dermatologiques du cystéate de zinc seul ou associé.

Les intéressantes propriétés pharmaceutiques du cystéate de zinc ont été mises en évidence par une série d'expérimentations dont les résultats sont résumés dans ce qui suit:

### TOXICITE AIGUE CHEZ LA SOURIS DU CB 619.

On a évalué la dose léthale 50% (DL 50) du CB 619 en solution dans l'eau bidistillée et administrée à des volumes variant de 0,2 à 0,3 ml/kg en observant les effets provoqués par l'administration pendant 14 jours.

Calculées suivant la méthode de MILLER et TAINTER, les DL 50 pour les animaux mâles et femelles ont été estimées à 0,10 ∓ 0,013 g/kg par voie intrapéritonéale et 1,65 ∓ 0,06 g/kg par voie orale.

Aux doses toxiques, les animaux s'isolent des leur cage et leur respiration devient difficile, ils présentent une pilo-érection et ont des difficultés à se mouvoir. On ne constate aucun phénomène de diarrhée.

### INDICE D'IRRITATION PRIMAIRE CUTANEE CHEZ LE LAPIN DU CB 619.

L'expérimentation a été effectuée suivant l'Arrêté Ministériel du 21 février 1982 précisant la méthode officielle pour la détermination de l'indice d'irritation primaire cutanée.

L'indice d'irritation primaire cutanée (IP) a été trouvé égal à 0,1666, soit nettement inférieur à 0,5, valeur au delà de laquelle un produit est classé comme "légèrement irritant". Le CB 619 est donc non irritant.

### TOLERANCE OCULAIRE CHEZ LE LAPIN DU CB 619.

L'expérimentation a été effectuée conformément aux recommandations de l'Arrêté Ministériel du 21 février 1984 précisant la méthode officielle pour la détermination de l'irritation oculaire.

En première approximation, le CB 619 a un indice d'irritation oculaire moyen (O.I.) maximum de 5,83 qui le classe parmi les produits très faiblement irritants. Ce résultat est confirmé par le fait que l'I.O. est inférieur au bout de 48 heures.

### DEGRE D'ALLERGENICITE DU CB 619 A L'AIDE DU TEST DE SENSIBILISATION SUR LE COBAYE.

L'expérimentation a été effectuée suivant la Fiche Technique n° 44 (J. Pharmacol. PARIS, 1978, 9, 1, 103-106) qui est conforme à la technique de MAGNUSSON et KLIGMANN.

L'absence de réaction spécifique de la peau des cobayes au CB 619 permet de conclure à sa non allergénicité vis-à-vis du test de sensibilisation chez le cobaye selon la technique de MAGNUSSON et KLIGMANN.

### PROPRIETES ANTISEBORRHEIQUES "IN VITRO" DU CB 619.

L'expérimentation a consisté à mesurer l'activité métabolique lipidique de la glande prépuciale du Rat. La synthèse lipidique de cette glande sébacée est jugée par incorporation d'acétate-¹⁴ C de sodium en qualité de précurseur marqué. On a étudié en parallèle le CB 619 et la carboxyméthylcystéïne (CMC) servant de substance de référence.
Le traitement par la CMC à 10⁻⁴M conduit à une inhibition de 5,9%
Le traitement par le CB 619 à 10⁻⁴M conduit à une inhibition de 33,7%
Le traitement par la CMC à 10⁻³M conduit à une inhibition de 24,5%
Le traitement par le CB 619 à 10⁻³M conduit à une inhibition de 41,8%
Le traitement par le CB 619 à 10⁻²M conduit à une inhibition de 51,5%
La CMC n'est pas assez soluble à 10⁻²M pour la conduite de l'expérience.

En conclusion, à la concentration de 10⁻²M le CB 619 permet d'obtenir une inhibition moyenne de la synthèse lipidique de plus de 50%, et à la concentration de 10⁻³ M, les deux molécules essayées sont actives, le CB 619 se montrant plus actif que la CMC.

### ACTIVITE CICATRISANTE CHEZ LE RAT DU CB 619.

L'expérimentation consiste à mesurer l'évolution de la surface d'une plaie par perte de substance.

Appliqué sur la plaie à la concentration de 3% dans une préparation à base de gel de carbopol à 2%, le CB 619 favorise la cicatrisation à la concentration de 3%, mais on ne retrouve plus cette activité à la concentration de 10%.

### CYTOTOXICITE DU CB 619 SUR LES CELLULES FIBROBLASTES HUMAINES.

Suivant la méthode de MOSMANN TIM (Journal of Immunological Methods, 65 (1983) 55-63), la dose non cytotoxique est de 50 »g/ml.

### ACTIVITE DU CB 619 SUR LA CROISSANCE CELLULAIRE.

On a comparé la vitesse de crois sance de deux lots de fibroblastes humains de même souche, l'un témoin, l'autre en contact avec une solution de CB 619 à une concentration déterminée. Les concentrations expérimentées ont été de 50, 5 et 1 »g/ml.

A 50 ug/ml, le CB 619 entraine un ralentissement de la multiplication cellulaire visible dès 48 heures, puis la mort des cellules.

Aux concentrations de 1 et 5 »g/ml, le produit augmente la multiplication cellulaire en fonction de la concentration. L'augmentation est significative au sixième jour pour la concentration à 1 »g/ml et elle est significative aux quatrième, sixième et huitième jours pour la concentration de 5 »g/ml.

Etant donné les intéressantes propriétés du cystéate de zinc, la présente invention concerne également ses applications cosmétologiques.

La présente invention concerne en outre les préparations pharmaceutiques et cosmétologiques qui contiennent du cystéate de zinc.

Pour les préparations à usage local, on choisira de préférence des concentrations en cystéate de zinc allant de 1 à 5%.

A titre d'exemple non limitatif, on confectionne un onguent reconstituant et antiséborrhéïque en ajoutant 3% de CB 619 à une base constituée de polyoxyéthylène glycol 59 g, polypropylène glycol 37 g, polysorbate 1g, eau de la Roche-Posay 3g.

## Revendications

1. A titre de produit industriel nouveau, sous forme anhydre ou hydratée, le cystéate de zinc de formule développée suivante : 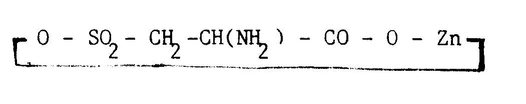 qui correspond à la formule brute suivante :
C₃H₅O₅NS Zn

2. Le cystéate de zinc suivant la revendication 1 caractérisé en ce qu'il est dihydraté suivant la formule suivante :
C₃H₅O₅NS Zn, 2H₂O

3. Le cystéate de zinc suivant l'une quelconque des revendications 1 ou 2 caractérisé en ce qu'il a été isolé sous forme de cristaux.

4. Les procédés de préparation du cystéate de zinc suivant l'une quelconque des revendications 1, 2 ou 3 caractérisés en ce que l'on fait réagir l'acide cystéïque ou l'un de ses sels sur un sel, un oxyde ou un hydroxyde de zinc.

5. Les procédés de préparation du cystéate de zinc suivant l'une quelconque des revendications 1, 2 ou 3 caractérisés en ce que l'on fait réagir le peroxyde de zinc sur la cystine ou la cystéïne en milieu légèrement acide.

6. Les procédés de préparation du cystéate de zinc suivant la revendication 4 caractérisés en ce que l'on fait réagir le sulfate de zinc avec l'acide cystéïque ou l'un de ses sels.

7. Les procédés de préparation du cystéate de zinc suivant la revendication 4 caractérisés en ce que l'on fait réagir le carbonate de zinc avec l'acide cystéïque ou l'un de ses sels.

8. Les procédés de préparation du cystéate de zinc suivant la revendication 4 caractérisés en ce que l'on fait réagir l'oxyde de zinc sur l'acide cystéïque ou l'un de ses sels.

9. Les procédés de préparation du cystéate de zinc suivant l'une quelconque des revendications 3, 4, 5, 6 ou 7 caractérisés en ce que la préparation a lieu en chauffant le mélange réactionnel.

10. Les médicaments et compositions pharmaceutiques caractérisés en ce qu'ils contiennent du cystéate de zinc suivant l'une quelconque des revendications 1, 2 ou 3.

11. Les compositions cosmétologiques caractérisées en ce qu'elles contiennent du cystéate de zinc suivant l'une des revendications 1, 2 ou 3.

12. Les traitements cosmétologiques caractérisés en ce que l'on utilise le cystéate de zinc suivant l'une quelconque des revendications 1, 2 ou 3.

13. Les médicaments ou compositions pharmaceutiques ou cosmétiques suivant l'une quelconque des revendications 10 ou 11 caractérisés en ce que leur indication est le traitement de la séborrhée.

14. Les médicaments ou compositions pharmaceutiques ou cosmétiques suivant l'une quelconque des revendications 10 ou 11 caractérisés en ce que leur indication est la cicatrisation des plaies de l'homme ou des animaux.

15. Les médicaments ou compositions pharmaceutiques ou cosmétiques suivant l'une quelconque des revendications 10 ou 11 caractérisés en ce que leur indication est l'amélioration de la croissance des cellules humaines ou animales.

## Claims

1. As new industrial product zinc cysteate in anhydrous or hydrated form, according to hereunder formula:

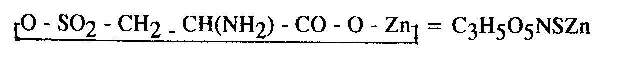

2. Zinc cysteate according to claim 1 when it is dihydrated according to hereunder formula:
C₃H₅O₅NSZn, 2H₂O

3. Zinc cysteate according to any claim belonging to the group consisting of claim 1 and 2 when it is isolated in crystals form.

4. A process to manufacture zinc cysteate according to any claim belonging to the group consisting of claims 1, 2 and 3 wherein cysteic acid or a cysteic salt reacts onto a zinc salt, zinc oxide, or zinc hydroxide.

5. A process to manufacture zinc cysteate according to any claim belonging to the group consisting of claims 1, 2 and 3 wherein zinc peroxide reacts onto cystine or cysteine in weak acidic solution.

6. A process to manufacture zinc cysteate according to claim 4 wherein zinc sulphate reacts onto cysteic acid or one of it salts.

7. A process to manufacture zinc cysteate according to claim 4 wherein zinc carbonate reacts onto cysteic acid or one of its salts.

8. A process to manufacture zinc cysteate according to claim 4 wherein zinc oxide reacts onto cysteic acid or one of its salts.

9. A process to manufacture zinc cysteate according to any claim belonging to the group consisting of claims 3, 4, 5, 6 and 7 wherein the reaction mixture is made warm.

10. Drugs and pharmaceutical compositions which contains zinc cysteate according to any claim belonging to the group consisting of claims 1, 2 and 3.

11. The cosmetical compositions which contains zinc cysteate according to any claim belonging to the group consisting of claims 1, 2 and 3.

12. The cosmetical treatments wherein zinc cysteate according to any claim belonging to the group consisting of claims 1, 2 and 3 is used.

13. Drugs or pharmaceutical or cosmetical compositions according to any claim belonging to the group consisting of claims 10 and 11 wherein the indication is the seborrhoea treatment.

14. Drugs or pharmaceutical or cosmetical compositions according to any claim belonging to the group consisting of claims 10 and 11 wherein the indication is cicatrization of wounds of human beings or animals.

15. Drugs or pharmaceutical or cosmetical compositions according to any claim belonging to the group consisting of claims 10 and 11 wherein the indication is growth improvement of cells of human beings or animals.

## Patentansprüche

1. Eigenschaft eines neuen Industrieergengnis, wasserfreies oder mit wasser verbundenes Cysteinsäure Zinksalz gemäsz der folgenden entwickelden Formel : 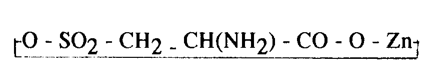 und der folgenden rohen Formel : C₃H₅O₅NSZn

2. Cysteinsäure Zinksalz nach Anspruch 1, dadurch gekennzeichnet, ist das dihydrat gemäsz der folgenden Formel: C₃H₅O₅NSZn, 2H₂O

3. Cysteinsäure Zinksalz nach Ansprüche 1 oder 2, dadurch gekennzeichnet, wird im cristallizert Form abgesondert wird.

4. Verfarhen zur Herstellung des Cysteinsäure Zinksalz nach einem der Ansprüche 1, 2 oder 3, dadurch gekennzeichnet, dasz Cysteinsäure oder eins seiner Hydroxyd umgesetzt wird.

5. Verfahren zur Herstellung des Cysteinsäure Zinksalz nach einem der Ansprüche 1, 2 oder 3, dadurch gekennzeichnet, dasz Zink Peroxyd mit Cystin oder Cystein in schwachem Säure Milieu umgesetzt wird.

6. Verfahrem zur Herstellung des Cysteinsäure Zinksalz nach Anspruch 4, dadurch gekennzeichnet, dasz Zinksulfat mit Cysteinsäure oder eins seiner Salze umgesetzt wird.

7. Verfahrem zur Herstellung des Cysteinsäure Zinksalz nach Anspruch 4, dadurch gekennzeichnet, dasz Zinkcarbonat mit Cysteinsäure oder eins seiner Salze umgesetzt wird.

8. Verfahrem zur Herstellung des Cysteinsäure Zinksalz nach Anspruch 4, dadurch gekennzeichnet, dasz Zinkoxyd mit Cysteinsäure oder eins seiner Salze umgesetzt wird.

9. Die Verfahrem zur Herstellung des Cysteinsäure Zincsalz nach einem der Ansprüche 3, 4, 5, 6 oder 7, dadurch gekennzeichnet, die reagierende Mischung erhitzt wird.

10. Arznei und pharmazeutische Zusammensetzungen, dadurch gekennzeichnet, dasz sie Cysteinsäure Zinksalz nach einem der Ansprüche 1, 2 oder 3 enthalten.

11. Kosmetische Zusammensetzungen, dadurch gekennzeichnet, dasz sie Cysteinsäure Zinksalz nach einem der Ansprüche 1, 2 oder 3 enthalten.

12. Kosmetische Behandlung, dadurch gekennzeichnet, dasz Cysteinsäure Zinksalz nach Ansprüche 1, 2 oder 3 benutzt wird.

13. Arznei oder pharmazeutische oder kosmetische Zusammensetzungen nach Ansprüche 10 oder 11, dadurch gekennzeichnet, die Ratschläge sind Seborrhea Behandlung.

14. Arznei oder pharmazeutische oder kosmetische Zusammensetzungen nach Ansprüche 10 oder 11, dadurch gekennzeichnet, die Ratschläge sind Vernärbung der Wunden der Menschen oder Tieren.

15. Arznei oder pharmazeutische oder kosmetische Zusammensetzungen nach Ansprüche 10 oder 11, dadurch gekennzeichnet, die Ratschläge sind Besserung der Entwicklung der Zellen des Menschen oder des Tieres.
